(19) Europäisches Patentamt
European Patent Office
Office européen des brevets

(11) Publication number: **0 502 464 A1**

# (12) EUROPEAN PATENT APPLICATION

(21) Application number: **92103593.7**

(51) Int. Cl.5: **C07D 477/00**, A61K 31/40

(22) Date of filing: **02.03.92**

(30) Priority: **07.03.91 GB 9104832**

(43) Date of publication of application:
**09.09.92 Bulletin 92/37**

(84) Designated Contracting States:
**AT BE CH DE DK ES FR GB GR IT LI LU MC NL PT SE**

(71) Applicant: **GLAXO S.p.A.**
**Via A. Fleming, 2**
**Verona(IT)**

(72) Inventor: **Biondi, Stefano, Glaxo SpA**
**Via Alessandro Fleming 2**
**I-37100 Verona(IT)**
Inventor: **Andreotti, Daniele,c/o Glaxo SpA**
**Via Alessandro Fleming 2**
**I-37100 Verona(IT)**
Inventor: **Rossi, Tino,c/o Glaxo SpA**
**Via Alessandro Fleming 2**
**I-37100 Verona(IT)**
Inventor: **Carlesso, Robert, c/o Glaxo SpA**
**Via Alessandro Fleming 2**
**I-37100 Verona(IT)**
Inventor: **Tarzia, Giorgio, c/o Glaxo SpA**
**Via Alessandro Fleming 2**
**I-37100 Verona(IT)**
Inventor: **Perboni, Alcide, c/o Glaxo SpA**
**Via Alessandro Fleming 2**
**I-37100 Verona(IT)**

(74) Representative: **Brewer, Christopher Laurence et al**
**Glaxo Holdings p.l.c. Glaxo House Berkeley Avenue**
**Greenford, Middlesex UB6 ONN(GB)**

## (54) Antibacterial condensed carbapenemes.

(57) Compounds of the formula (I)

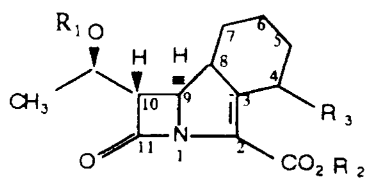

(I)

in which $R_1$ represents a hydrogen atom or a hydroxyl protecting group;
$R_2$ represents a hydrogen atom, a carboxyl protecting group or a cation derived from an inorganic base or an organic base;
$R_3$ represents the group $O(CH_2)_nR_4$ in which n is an integer from 2 to 6 and $R_4$ represents a halogen atom or a hydroxy, $C_{1-3}$alkoxy, $C_{1-3}$ alkythio, azido, cyano, nitro or quaternary ammonium group $\overset{+}{N}R_5R_6R_7X^-$wherein $R_5$ and $R_6$ independently represent a $C_{1-3}$alkyl group, $R_7$ is a $C_{1-2}$ alkyl group and $X^-$ is a pharmaceutically acceptable anion and metabolically labile esters, salts and solvates thereof, and processes for their production.

EP 0 502 464 A1

This invention relates to heterocyclic derivatives having antibacterial activity, to processes for their preparation, to compositions containing them, and to their use in medicine.

Thus the present invention provides compounds of the general formula (I)

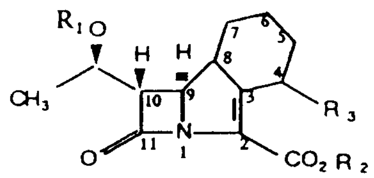

(I)

in which $R_1$ represents a hydrogen atom or a hydroxyl protecting group;

$R_2$ represents a hydrogen atom, a carboxyl protecting group or a cation derived from an inorganic base or an organic base;

$R_3$ represents the group $O(CH_2)_nR_4$ in which n is an integer from 2 to 6 and $R_4$ represents a halogen atom or a hydroxy, $C_{1-3}$alkoxy, $C_{1-3}$ alkythio, azido, cyano, nitro or quaternary ammonium group $\overset{+}{N}R_5R_6R_7X^-$wherein $R_5$ and $R_6$ independently represent a $C_{1-3}$alkyl group, $R_7$ is a $C_{1-2}$ alkyl group and $X^-$ is a pharmaceutically acceptable anion and metabolically labile esters, salts and solvates thereof.

In addition to the fixed stereochemical arrangement as defined in formula (I) the molecule contains a further asymmetric carbon atom at the 8-position, and another at the 4-position. It will be appreciated that all stereoisomers including mixtures thereof arising from these additional asymmetric centres, are within the scope of the compounds of formula (I).

The compounds of formula (I) are antibacterial agents and/or of use as intermediates for the preparation of other active compounds within the general formula (I). Compounds wherein $R_1$ represents a hydroxyl protecting group and/or wherein $R_2$ represents a carboxyl protecting group are in general intermediates for the preparation of other compounds of formula (I).

Suitable hydroxyl protecting groups $R_1$ and carboxyl protecting groups $R_2$ include those which may be removed by hydrolysis under buffered conditions or under non-aqueous conditions.

When the group $OR_1$ is a protected hydroxyl group this is conveniently an ether or an acyloxy group. Examples of particularly suitable ethers include those in which $R_1$ is a hydrocarbylsilyl group such as trialkylsilyl, e.g. trimethylsilyl or t-butyldimethylsilyl. When the group $OR_1$ represents an acyloxy group then examples of suitable groups $R_1$ includes alkanoyl e.g. acetyl, pivaloyl; alkenoyl e.g. allylcarbonyl; aroyl e.g. p-nitrobenzoyl; alkoxycarbonyl e.g. t-butoxycarbonyl; haloalkoxycarbonyl e.g. 2,2,2-trichloroethoxycarbonyl, or 1,1,1-trichloro-2-methyl-2-propoxycarbonyl; aralkyloxycarbonyl e.g. benzyloxycarbonyl or P-nitrobenzyloxycarbonyl; or alkenyloxycarbonyl e.g. allyloxycarbonyl.

A particularly convenient protecting group $R_1$ is t-butyldimethylsilyl.

Examples of suitable carboxyl protecting groups include arylmethyl groups such as benzyl, p-nitrobenzyl or trityl, or alkenyl groups such as allyl or substituted allyl, t-butyl, haloalkyl e.g. trichloroethyl or trialkylsilylalkyl e.g. trimethylsilylethyl. Preferred protecting groups $R_2$ include arylmethyl e.g. benzyl or allyl.

Particularly useful compounds of formula (I) for use in medicine as antibacterial agents are those in which the group $R_1$ represents a hydrogen atom and $R_2$ represents a hydrogen atom or a physiologically acceptable cation, or an internal salt thereof. These compounds exhibit antibacterial activity against a wide range of gram positive and gram negative, aerobic and anaerobic pathogenic microorganisms.

Where $R_2$ is a physiologically acceptable cation, suitable cations include those of alkali metals (e.g. sodium or potassium), alkaline earth metals (e.g. calcium), amino acids (e.g. lysine and arginine) and organic bases (e.g. procaine, phenylbenzylamine, dibenzylethylenediamine, ethanolamine, diethanolamine, and N-methyl glucosamine).

Where $R_2$ is a cation that is not physiologically acceptable then such compounds may be useful as intermediates for the preparation and/or isolation of other compounds of the invention.

When $R_4$ represents the group $\overset{+}{N}R_5R_6R_7$ then the group $CO_2R_2$ may be the anion $CO^-{}_2$ and such internal salts are also within the scope of the invention.

The general formula (I) as drawn includes at least 4 stereoisomers and mixtures thereof and these may be represented by the formulae (1a, 1b, 1c and 1d).

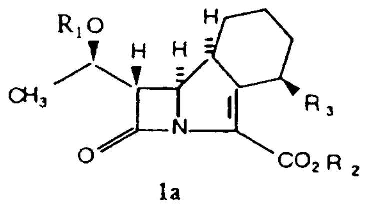

1a

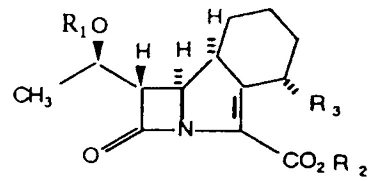

1b

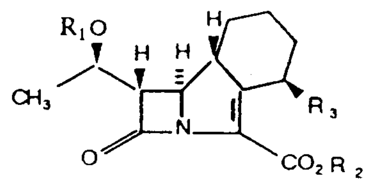

1c

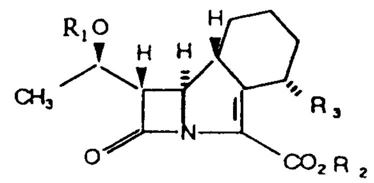

1d

The wedge shaped bond ◄ indicates that the bond is above the plane of the paper. The broken bond ıııı indicates that the bond is below the plane of the paper.

The configuration shown for the carbon atom at the 8-position in formulae 1a and 1b is hereinafter referred to as the β configuration and in formulae 1c and 1d as the α configuration.

The configuration shown for the carbon at the 4 position in formulae 1b and 1d is hereinafter referred to as the α confirguation and in formulae 1a and 1c as the β configuration.

In general, in the specific compounds named below, the β-configuration at the 8-position corresponds to the S isomer and the β-configuration at the 4-position to the R isomer. The α configuration at the 8-position corresponds to the R isomer and the α-configuration at the 4-position corresponds to the S isomer. The assignment of the R or S configuration at the 4- and 8- positions have been made according to the rules of Cahn. Ingold and Prelog, Experientia 1956, 12, 81.

When $R_4$ represents a halogen atom this may be for example a fluorine, chlorine, bromine or iodine atom.

The groups $R_5$ and $R_6$ independently represent a $C_{1-3}$alkyl group and thus may be for example a methyl ethyl or propyl group. $R_7$ may be for example a methyl or ethyl group. The group $X^-$ is conveniently a halide ion such as $Cl^-$, $Br^-$ or $I^-$.

A preferred group of compounds of formula I are those in which the carbon atom at the 8- position is in the β configuration. Within this group those compounds in which the carbon atom at the 4-position is in the α configuration are particularly preferred.

A further preferred group of compounds of the invention are those in which n is an integer from 2 to 3. Within this group those wherein $R_4$ represents halogen e.g. fluorine or iodine, hydroxy, methoxy, methylthio, azido cyano, or trimethylammonium group are particularly preferred.

A particularly preferred group of compounds of formula (I) are those in which the carbon atom at the 8-position is in the β configuration and and the carbon atom at the 4- position in the α configuration, $R_1$ represents a hydrogen atom, $R_2$ represents a hydrogen atom or a physiologically acceptable cation and $R_3$ represents the group $O(CH_2)_nR_4$ wherein n is 2 or 3 and $R_4$ represents fluoro, iodo, azido, hydroxy, methoxy, cyano or trimethylammonium and X is halide ion, and salts thereof.

Specific preferred compounds include (4S,8S,9R,10S,12R)-4-(2-hydroxyethoxy)-10-(1-hydroxyethyl)-11-oxo-1-azatricyclo [7.2.0.$0^{3,8}$] undec-2-ene-2-carboxylic acid and salts thereof e.g. sodium or potassium salt.

(4S,8S,9R,10S,12R)-4-(2-methoxyethoxy)-10-(1-hydroxyethyl)-11-oxo-1-azatricyclo[7.2.0.$0^{3,8}$]undec-2-ene-2-carboxylic acid and salts thereof e.g. potassium or sodium salt.

(4S,8S,9R,10S,12R)-4-(2-azidoethoxy)-10-(1-hydroxyethyl)-11-oxo-1-azatricyclo[7.2.0.$0^{3,8}$]undec-2-ene-2-carboxylic acid and salts thereof e.g. potassium or sodium salt.

(4S,8S,9R,10S,12R)-4(2-iodoethoxy)-10-(-1-hydroxyethyl)-11-oxo-1-azatricyclo[7.2.0.$0^{3,8}$]undec-2-ene-2-carboxylic acid and salts thereof e.g. potassium or sodium salt.

(4S,8S,9R,10S,12R)-4-[2-(trimethylammonium)-ethoxy]-10-(1-hydroxyethyl)-11-oxo-1-azatricyclo[7.2.0.2.$^{3,8}$]-undec-2-ene-2-carboxylate.

(4S,8S,9R,10S,12R)-4(2-fluoroethoxy)-10-(1-hydroxyethyl)-11-oxo-1-azatricyclo[7.2.0.$0^{3,8}$]undec-2-ene-2-carboxylic acid and salts thereof e.g. potassium or sodium salt.

(4S,8S,9R,10S,12R)-4(2-cyanoethoxy)-10-(1-hydroxyethyl)-11-oxo-1-azatricyclo[7.2.0.$0^{3,8}$]undec-2-ene-2-carboxylic acid and salts thereof e.g. potassium or sodium salt.

(4S,8S,9R,10S,12R)-4(3-hydroxypropoxy)-10-(1-hydroxyethyl)-11-oxo-1-azatricyclo[7.2.0.$0^{3,8}$]undec-2-ene-2-carboxylic acid and salts thereof e.g. potassium or sodium salt.

(4S,8S,9R,10S,12R)-4(3-azidopropoxy)-10-(1-hydroxyethyl)-11-oxo-1-azatricyclo[7.2.0.$0^{3,8}$]undec-2-ene-2-carboxylic acid and salts thereof e.g. potassium or sodium salt.

Compounds according to the invention not only exhibit a broad spectrum of antibacterial activity against a wide range of pathogenic microorganisms but also have a very high resistance to all $\beta$-lactamases. Compounds of the invention are also relatively stable to renal dehydropeptidase.

Compounds of the invention have been found to exhibit useful levels of activity against strains of Staphylococcus aureus, Streptococcus faecalis, Escherichia coli, Pseudomonas aeruginosa, Clostridium perfringens and Bacteriodes fragilis.

The compounds of the invention may therefore be used for treating a variety of diseases caused by pathogenic bacteria in human beings and animals.

Thus, according to another aspect of the present invention, we provide a compound of formula (I) for use in the therapy or prophylaxis of systemic or topical bacterial infections in a human or animal subject.

According to a further aspect of the invention we provide the use of a compound of formula (I) for the manufacture of a therapeutic agent for the treatment of systemic or topical bacterial infections in a human or animal body.

According to a yet further aspect of the invention we provide a method of treatment of the human or non-human animal body to combat bacterial infections which method comprises administering to the body an effective amount of a compound of formula (I).

The compounds of the invention may be formulated for administration in any convenient way for use in human or veterinary medicine and the invention therefore includes within its scope pharmaceutical compositions comprising a compound of the invention adapted for use in human or veterinary medicine. Such compositions may be presented for use in conventional manner with the aid of one or more suitable carriers or excipients. The compositions of the invention include those in a form especially formulated for parenteral, oral, buccal, rectal, topical, implant, ophthalmic, nasal or genito-urinary use.

The compounds according to the invention may be formulated for use in human or veterinary medicine by injection (e.g. by intravenous bolus injection or infusion or via intramuscular, subcutaneous or intrathecal routes) and may be presented in unit dose form, in ampoules, or other unit-dose containers, or in multi-dose containers, if necessary with an added preservative. The compositions for injection may be in the form of suspensions, solutions, or emulsions, in oily or aqueous vehicles, and may contain formulatory agents such as suspending, stabilising, solubilising and/or dispersing agents. Alternatively the active ingredient may be in sterile powder form for reconstitution with a suitable vehicle, e.g. sterile, pyrogen-free water, before use.

The compounds of the invention may also be presented for human or veterinary use in a form suitable for oral or buccal administration, for example in the form of solutions, gels, syrups, mouth washes or suspensions, or a dry powder for constitution with water or other suitable vehicle before use, optionally with flavouring and colouring agents. Solid compositions such as tablets, capsules, lozenges, pastilles, pills, boluses, powder, pastes, granules, bullets or premix preparations may also be used. Solid and liquid compositions for oral use may be prepared according to methods well known in the art. Such compositions may also contain one or more pharmaceutically acceptable carriers and excipients which may be in solid or liquid form.

The compounds of the invention may also be administered orally in veterinary medicine in the form of a liquid drench such as a solution, suspension or dispersion of the active ingredient together with a pharmaceutically acceptable carrier or excipient.

The compounds of the invention may also, for example, be formulated as suppositories e.g. containing conventional suppository bases for use in human or veterinary medicine or as pessaries e.g. containing conventional pessary bases.

The compounds according to the invention may be formulated for topical administration, for use in human and veterinary medicine, in the form of ointments, creams, gels, lotions, shampoos, powders, (including spray powders), pessaries, tampons, sprays, dips, aerosols, drops (e.g. eye ear or nose drops) or pour-ons.

Aerosol sprays are conveniently delivered from pressurised packs, with the use of a suitable propellant, eg dichlorodifluoromethane, trichlorofluoromethane, dichlorotetrafluoroethane, carbon dioxide or other suitable gas.

For topical administration by inhalation the compounds according to the invention may be delivered for use in human or veterinary medicine via a nebuliser.

The pharmaceutical compositions for topical administration may also contain other active ingredients such as corticosteroids or antifungals as appropriate.

The compositions may contain from 0.01-99% of the active material. For topical administration, for example, the composition will generally contain from 0.01-10%, more preferably 0.01-1% of the active material.

For systemic administration the daily dose as employed for adult human treatment will range from 5-100mg/kg body weight, preferably 10-60mg/kg body weight, which may be administered in 1 to 4 daily doses, for example, depending on the route of administration and the condition of the patient. When the composition comprises dosage units, each unit will preferably contain 200mg to 1g of active ingredient.

The duration of treatment will be dictated by the rate of response rather than by arbitrary numbers of days.

The compounds of formula (I) in which $R_4$ is a hydroxy, fluoro, alkoxy, alkythio, cyano or nitro group may be prepared by the cyclisation of a compound of formula (II)

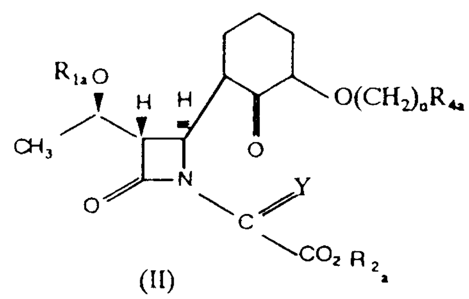

(II)

in which the group $R_{4a}$ has the meansings defined above for $R_4$ or is a group convertible thereto, n is as defined in formula (I) and Y is an oxygen atom or a phosphine group, and the groups $R_{1a}$ and $R_{2a}$ are hydroxy and carboxyl protecting groups as defined for $R_1$ and $R_2$ and if required or desired subjecting the resulting compound prior to or subsequent to any separation into its stereochemical isomers, to one or more of the following operations

(a) removal of one or more protecting groups.

(b) conversion of the group $R_{4a}$ into the group $R_4$.

(c) conversion of a compound in which $R_2$ is a hydrogen atom or a carboxyl protecting group into a salt of an inorganic or organic base.

The cyclisation of a compound of formula (II) in which Y is oxygen is conveniently carried out by heating in the presence of an organic phosphite. The reaction is preferably carried out in a solvent or mixture of solvents at a temperature within the range 60-200$^0$. Suitable solvents include hydrocarbons with an appropriate boiling point, for example aromatic hydrocarbons, such as toluene or xylene.

Suitable organic phosphites include acyclic and cyclic trialkylphosphites, triarylphosphites and mixed alkylarylphosphites. Particularly useful organic phosphites are the trialkylphosphites e.g. triethylphosphite or trimethylphosphite.

The cyclisation of a compound of formula (II) in which Y is a phosphine grouping is preferably carried out in a solvent at a temperature between 40-200$^0$C. Suitable solvents include hydrocarbons such as aromatic hydrocarbons, for example xylene or toluene, aliphatic hydrocarbons and halogenated hydrocarbons such as dichloromethane, chloroform and trichloroethane. Examples of suitable phosphine groups are triarylphosphines e.g. triphenyl phosphine, or trialkylphosphines e.g. tri-t-butylphosphine.

The hydroxyl and carboxyl protecting groups $R_{1a}$ and $R_{2a}$ may be removed by conventional procedures and in any order. More preferably however the hydroxyl protecting group $R_{1a}$ is removed prior to the removal of the carboxyl protecting group. Such removal of the protecting groups is a further feature of the invention.

The hydroxyl protecting groups may be removed by well known standard procedures such as those described in Protective Groups in Organic Chemistry, pages 46-119, Edited by J F W McOmie (Plenum Press, 1973). For example when $R_{1a}$ is a t-butyldimethylsilyl group, this may be removed by treatment with tetrabutylammonium fluoride and acetic acid. This process is conveniently carried out in a solvent such as tetrahydrofuran. Similarly when $R_{1a}$ is a trichloroethoxycarbonyl group this may be removed by treatment with zinc and acetic acid.

The carboxyl protecting group $R_{2a}$ may also be removed by standard processes such as those described in Protective Groups in Organic Chemistry, pages 192-210, Edited by J F W McOmie (Plenum

Press 1973). For example when $R_{2a}$ represents an arylmethyl group this may be removed by conventional procedures using hydrogen and a metal catalyst e.g. palladium. When the group $R_{2a}$ represents an allyl or substituted allyl group then this is preferably removed by treatment with an allyl acceptor in the presence of tetrakis(triphenylphosphine) palladium and optionally in the presence of triphenylphosphine. Suitable allyl acceptors include sterically hindered amines such as tertbutylamine, cyclic secondary amines such as morpholine or thiomorpholine, tertiary amines such as triethylamine, aliphatic or cycloaliphatic $\beta$-dicarbonyl compounds such as acetylacetone, ethyl acetoacetate or dimedone, or alkanoic acids or alkali metal salts thereof such as acetic acid, propionic acid or 2-ethyl hexanoic acid or the potassium or sodium salt thereof.

A particularly useful allyl acceptor is 2-ethylhexanoic acid and more especially the sodium or potassium salts thereof.

The reaction is preferably carried out in an inert solvent such as an ether e.g. diethyl ether or tetrahydrofuran, an alkanol e.g. ethanol, an ester e.g. ethyl acetate or a halohydrocarbon e.g. methylene chloride, or mixtures thereof. The reaction is conveniently carried out in the temperature range $0^{0}$-$40^{0}$ more particularly at room temperature.

Compounds of the invention in which the group $R_2$ is a physiologically acceptable cation may be prepared from compounds of the invention in which $R_2$ is hydrogen by treatment with a suitable base. Conveniently the salt is formed in solution and then if required precipitated by the addition of a non-solvent e.g. a non polar aprotic solvent. Alternatively the sodium or potassium salt may be prepared by treating a solution of a compound of formula (I) in which $R_2$ represents a hydrogen atom with a solution of sodium or potassium 2-ethylhexanoate in a non-polar solvent such as diethyl ether.

For the preparation of compounds of formula I in which $R_4$ represents a hydroxyl group the cyclisation reaction is conveniently carried out using an intermediate of formula (II) in which $R_{4a}$ is a protected hydroxyl group. Suitable protected hydroxyl groups include trihydrocarbyl silyl ethers such as the trimethylsilyl or t-butyldimethylsilyl ether. The hydroxyl protecting group may then be removed at any subsequent stage in the synthesis, for example at the same time as the removal of the hydroxyl protecting group $R_{1a}$.

Compounds of formula (I) may be converted into other compounds of formula (I). Thus compounds of formula (I) wherein the groups $R_1$ is a hydroxyl protecting group and $R_2$ is a carboxyl protecting group and $R_4$ is a halogen atom may be prepared from the corresponding compound of formula (I) in which $R_4$ is a hydroxyl group using conventional procedures. For example by reaction with a phosphorous trihalide or phosphourus pentahalide. Alternatively the alcohol may be reacted with an alkyl or arylsulphonyl halide in the presence of a base, and the sulphonate ester thus formed treated with the appropriate alkali metal halide e.g. sodium iodide in a suitable solvent such as acetone to yield the required halide. If desired the protecting groups $R_1$ and $R_2$ may then be removed by conventional means.

Compounds of formula (I) wherein $R_2$ is a carboxyl protecting group, and $R_1$ is a hydrogen atom or a hydroxyl protecting group and $R_4$ is a trialkylammonium group $-N^{+}-R_5R_6R_7$, may be prepared from the corresponding compound in which $R_4$ is a halogen atom, for example iodine, by reaction with the teriary amine $NR_5R_6R_7$. The reaction is preferably carried out in a solvent such acetonitrile and optionally with heating. If desired the carboxyl, protecting group may then be removed by conventional means.

Compounds of formula (I) in which $R_1$ and $R_2$ represents hydroxyl and carboxyl protecting groups respectively and $R_4$ represents a cyano group may be prepared from the corresponding compound in which $R_4$ represents a halogen atom such as iodine, by reaction with an alkali metal nitrile such as sodium or potassium nitrile in a suitable solvent. If desired the hydroxyl and carboxyl protecting groups $R_1$ and $R_2$ respectively may then be removed to give compounds of formula (I) in which $R_1$ represents a hydrogen atom and $R_2$ represents a hydrogen atom or a cation.

Compounds of formula (I) in which $R_1$ and $R_2$ represent hydroxyl and carboxyl protecting groups respectively and $R_4$ represents an azido group may be prepared from the corresponding group in which $R_4$ represents hydroxy by reaction with sodium azide in the presence of triphenylphosphine and carbon tetrabromide. The reaction is carried out in a polar aprotic solvent such as dimethylformamide and preferably at a temperature between $-10^{0}$ to $10^{0}$.

Compounds of formula (II) in which Y = 0 may be prepared by treating a compound of formula (III) in which the group $R_{1a}$ and $R3_a$ have the meanings given above with an activated derivative of the acid (IV) in which $R_{2a}$ has the meanings defined above.

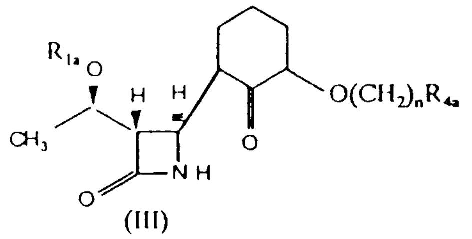

(III)

$HOOCCO_2R_{2a}$

(IV)

Suitable activated derivatives of the acid (IV) includes the corresponding acid halides e.g. acid chloride.

When the acid halide is used as the activated derivative of the acid (IV) then the reaction is preferably carried out in the presence of an acid acceptor such as a tertiary organic base for example pyridine or a trialkylamine in an aprotic solvent such as dichloromethane.

The compound of formula (II) in which Y is a phosphine group may be prepared by treating the intermediate (V) in which L is a leaving group such as a halogen e.g. chlorine

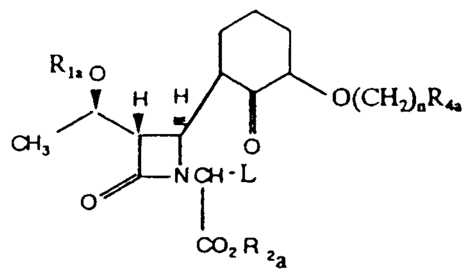

(V)

with the corresponding phosphine e.g. triphenylphosphine in the presence of a base. The reaction is conveniently carried out in a solvent such as dioxan in the presence of a tertiary organic base, e.g. 2,6 lutidine. The compounds of formula (II) are novel compounds and as such form a further asepct of the invention.

The compounds of formula (V) may be prepared from the corresponding hydroxy derivative (VI) by conventional means for converting hydroxyl groups into leaving groups.

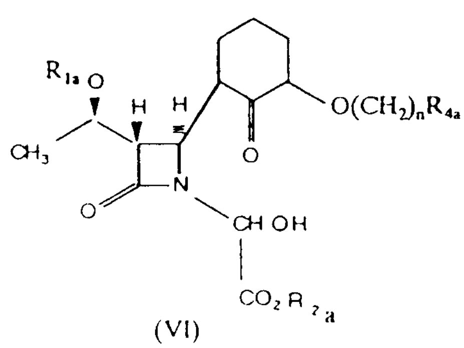

(VI)

Thus for example a compound of formula (V) in which L is a chlorine atom may be prepared by treating a compound of formula (VI) with thionyl chloride in an aprotic solvent such as dioxan or tetrahydrofuran and in the presence of a tertiary organic base e.g. 2,6-lutidine. Compounds of formula (VI) may be prepared from the reaction of a compound of formula (III) with glyoxylic ester (VII; $CHOCO_2R_{2a}$) preferably in the form of its hydrate or hemiacetal. The reaction is preferably carried out in an aprotic solvent such as toluene and in the presence of an activated molecular sieve. Compounds of formula (VI) may also be prepared by reduction of a compounds of formula (II) in which Y = O. Suitable reducing agents include zinc/acetic acid.

Alternatively compounds of formula (II) in which Y = O, may be prepared by oxidation of a compound of formula (VI), using for example manganese dioxide.

Compounds of formula (III) may be prepared by treating the azetidinone (VIII) with the enolate ion of the

ketone (IX).

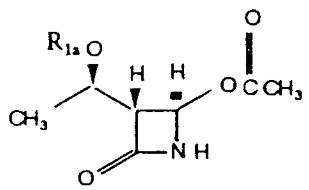

(VIII)

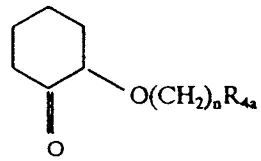

(IX)

The reaction is preferably carried out at a low temperature e.g. -78°C in a solvent such as tetrahydrofuran.

The enolate ion of the ketone (IX) is conveniently generated in situ by treatment with a suitable base such as lithium bis(trimethyl silyl)amide.

Compounds of formula (III) may also be prepared by reduction of the compound of formula (X) in which $R_{1a}$ and $R_{4a}$ are as defined above.

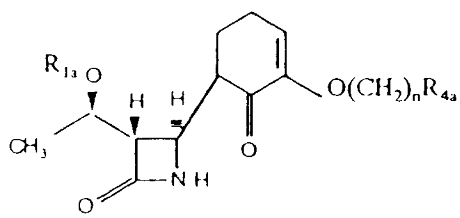

(X)

The reduction may be effected using hydrogen and a metal catalyst e.g. palladium on a suitable support e.g. carbon or alumina. The reaction is carried out in a solvent such as an ester e.g. ethyl acetate.

The compound of formula (X) may be prepared from the reaction of the azetidinone (VIII) with the ketone (XI) using the conditions described above for preparing compounds of formula (III) from the ketone (IX).

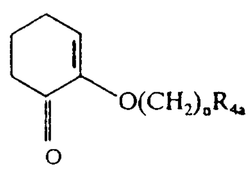

(XI)

Compounds of formula (III) may also be prepared by oxidation of the alcohol of formula (XIII)

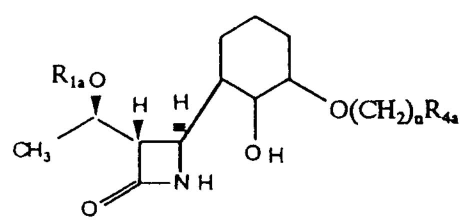

(XIII)

in which the groups $R_{1a}$ and $R_{4a}$ have the meanings defined above. The oxidation may be carried out using conventional oxidising agents known in the art for converting a secondary alcohol such as a cyclohexanol

into a ketone such as a cyclohexanone. Thus for example the oxidation may be carried out using pyridinium chlorochromate or oxalyl chloride and dimethylsulphoxide. The reactions are preferably carried out in a solvent such as methylene chloride.

The alcohol (XIII) may be prepared by reduction of the $\alpha$-$\beta$ unsaturated ketone (X). This reduction is conveniently carried out in a two stage reaction. The first stage is the reduction of the ketone to the alcohol using a suitable metal hydride such as sodium borohydride. The resultant $\alpha$-$\beta$ unsaturated alcohol is then reduced to the required alcohol (XIV) using hydrogen and a metal catalyst as described above for the preparation of the ketone (III) from the $\alpha$-$\beta$ unsaturated ketone (X).

The alcohol of formula (XIII) may be prepared by reacting the corresponding epoxide (XIV) with the corresponding alcohol $HO(CH_2)_nR_{4a}$ in the presence of an acid catalyst such as p-toluene sulphonic acid.

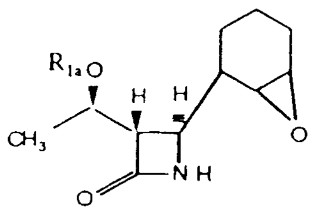

(XIV)

In any of the formulae (I) to (XIV) shown above when there is an asymmetric carbon atom and no specific configuration is shown then the formula includes all possible configurations.

Specific stereoisomers of the compounds of formula (I) as defined in formulae 1a, 1b, 1c and 1d, essentially free of the other stereoisomers may be prepared by using the general processes described above starting with the appropriate stereoisomer of formula (III).

The processes described above for preparing the compounds of formula (III) will in general give a mixture of stereoisomers.

The individual stereoisomers of the compounds of formula (III) may be separated from each other by conventional techniques such as fractional crystallisation or more particularly by column chromatography, using for example a silica column, as illustrated in the relevant examples.

The compounds of formulae (III), (X) and (XIII) are novel compounds and these compounds and the individual stereoisomers thereof form a further aspect of the invention.

Alternatively the synthesis may be carried out starting with a mixture of 2 or more stereoisomers of formula (III) and the required specific stereoisomer separated at by conventional techniques at another stage in the synthesis. Thus the compounds may be separated by fractional crystallisation and or column chromatography.

In the synthesis of compounds of formula (I) or the intermediates therefore it may be necessary to protect functional groupings within the group $R_3$. Such protection and deprotection steps are conventional and are within the scope of the invention. For example when the group $R_3$ contains a hydroxyl groups then it may be desirable to protect these during the synthesis using conventional hydroxyl protecting groups.

the compounds of formulae (VIII), (IX), (XI) and (XIV) are either known compounds or may be prepared by analogous methods to those used for known compounds.

In order that the invention may be more fully understood the following examples are given by way of illustration only.

In the Preparations and Examples, unless otherwise stated:

Melting points (m.p.) were determined on a Gallenkamp m.p. apparatus and are uncorrected. All temperatures refer to $^{0}C$.

Infrared spectra were measured in chloroform-$d_1$ solutions on a FT-IR instrument. Proton Magnetic Resonance (1H-NMR) spectra were recorded at 300 MHz as solutions in chloroform-$d_1$. Chemical shifts are reported in ppm downfield ($\delta$) from $Me_4Si$, used as an internal standard, and are assigned as singlets (s), doublets (d), doublet of doublets (dd) or multiplets (m).

Column chromatography was carried out over silica gel (Merck AG Darmstadt, Germany).

Solutions were dried over anhydrous sodium sulphate.

"Petrol" refers to petroleum ether, b.p. 40-60$^{0}$C.

Methylene chloride was redistilled over calcium hydride; tetrahydrofuran was redistilled over sodium; ethyl ether was redistilled over sodium; xylene was redistilled over phosphorus pentoxide and ethyl acetate was dried over activated molecular sieves.

The following abbreviations are used in the tables and text.
EA = ethyl acetate, CH = cyclohexane, P = petroleum ether 40-60°C,
THF = tetrahydrofuran, MC = methylene chloride, EE = ethyl ether.
Tlc refers to thin layer chromatography on silica plates.

Intermediate 1

(3S,4R)-3-[(R)-1-(t-Butyldimethylsilyoxy)ethyl-4-((2'S)-2'(6'S)-6'(2-methoxyethoxy)-1-oxocyclohexyl)]azetidin-2-one (1a) and (3S,4R)-3-[(R)-1-(t-Butyldimethylsilyoxy)ethyl-4-((2'R)2'(6'R)-6'(2-methoxyethoxy)-1-oxocyclohexyl)]azetidin-2-one (1b)

n-Butyl lithium (16ml of a 2.5M solution in hexane) was added dropwise to a solution of 2,2,6,6-tetramethylpiperidine (5.65g) in dry tetrahydrofuran (50ml) stirred at 0C under nitrogen. After stirring at 0C for 30 min the mixture was cooled to -78C, 2-(2-methoxyethoxy)cyclohexanone (4g) was then added dropwise at a rate such that the reaction temperature remained below -70C. The reaction mixture was stirred at -78C for 30 min and then (3S,4R)-4-Acetoxy-3-[(R)-1-(t-butyldimethylsilyoxy)ethyl]-2-azetidinone (3.33g) was added as a solid. Immediately after the formation of a homogeneous solution. The reaction was quenched by the addition of saturated ammonium chloride (100ml). The resulting reaction mixture was extracted with ethyl acetate (3 x100ml). The combined organic extracts were washed with a cold solution of 5% hydrochloric acid (100ml) and with a saturated solution of sodium hydrogen carbonate (100ml) dried, and evaporated under reduced pressure to give a yellow oil. Purification by flash chromatography (gradient elution with mixtures of cyclohexane/ethyl acetate, form 95/5 to 50/50 gave 1/1 mixture of the title compounds (Rf = $SiO_2$, CH/EA 2/8) as colourless oil
IR (V $cm^{-1}$) 1757($\beta$-lactam), 1717(carbonyl)
$^1$H-NMR ($\delta$ ppm) 6.03(bs), 5.82(bs), 4.17(m), 3.98(m), 3.69(m), 3.64-3.3(m), 3.36(s), 3.35(s), 3.15(m), 2.88-(m), 2.74(m), 2.27(m), 2.15-1.9(m), 1.67(m), 1.24(d), 1.21(d), 0.87(s), 0.072(s), 0.061(s).

Intermediate 2

2-(2-benzyloxyethoxy)-cyclohexanone

A mixture of dimeric 2-hydroxycyclohexanone (13.7g), 2-benzyloxyethanol (20g) and p-toluensulphonic acid (2g) were dissolved in xylene (500ml) in a round bottom flask fitted with a Dean Stark apparatus and reluxed for 10hrs. The resulting solution was cooled, washed with sodium hydrogen carbonate (3x50ml) dried and concentrated under reduced pressure. The crude oil was then pruified by flash chromatography using cyclohexane/ethyl acetate 60/40 as eluant yielding 20g of the title compound (Rf = 0.5).
IR, $CDCl_3$ ($cm^{-1}$): 1722 (C = O), 1603(C = C).
$^1$H-NM, 300 MHz, $CDCl_3$, 7.32(m), 4.55(dd), 3.92(m), 3.83(m), 3.64(m), 3.60(m), 2.48(m), 2.24(m), 1.93(m), 1.8-1.55(m).

Intermediate 3

(3S,4R)-3-[(R)-1-(t-butyldimethylsilyloxy)ethyl]-4-[(R)-2'-[(S)-6'-(2-benzyloxyethoxy)-1'-oxocyclohexyl]]-azetidin-2-one

2,2,6,6-tetramethylpiperidine (12.7g) was added dropped to a solution of n-butyllithium 2.5M in hexane (33ml) in tetrahydrofuran (150ml) at -70° under a nitrogen atmosphere. The reaction mixture was then warmed to 10°, recooled to -70° and intermediate 3 (18.72g) was slowly added maintaining the temperature below -70°. After the addition was completed, the solution was maintained at that temperature for 15 min and (3R,4R)-4-acetoxy-3-((R)-1-t-butyldimethylsilyloxy)-2-azetidinone (11.48g), dissolved in THF (200ml) was added over 30 mins maintaining the temperature below -70°. The reaction was quenched after 5 minutes using a mixture of ammonium chloride (100 ml saturated solution) and hydrochloric acid (200 ml 10% solution) and extracted with ethyl acetate. The organic layer was washed with brine, dried, concentrated under reduced pressure and purified by flash chromatography using cyclohexane/ethyl acetate 85/15 to 30/70 as eluant to give the title compound (2.2g, Rf = 0.65).
IR, $\nu_{max}$ ($CDCl_3$) ($cm^{-1}$): 3418(NH), 1757(C = O lactam), 1718 (C = O), 1603 (C = O).
$^1$H-NMR 300 MHz ($CDCl_3$) 7.32(m), 5.71 (s broad), 4.56 (s + m), 4.18(m), 3.99(m), 3.73(m), 3.6-3.5(m), 3.15-(m), 2.87(dd), 2.30(m), 2.10(m), 1.80-1.50(m), 1.19(d), 0.86(s), 0.07(s + s);

Intermediate 4

(3S,4R)-3-[(R)-1-(t-butyldimethylsilyloxy)ethyl]-4-[(R)-2'-[(S)-6'-(2-hydroxyethoxy)-1'-oxocyclohexyl]]azetidin-2-one

10%Pd/C (300mg) intermediate 3 (1g) and ethyl acetate (30ml), were placed in a flask under a hydrogen pressure of 1 atmosphere and stirred for 6hrs. The reaction mixture was then filtred through a celite pad and washed with ethyl acetate several times yielding the title compound as a white solid (1g., RF - 0.45 ethylacetate/cyclohexane 7/3)
IR, $CDCl_3$ ($cm^{-1}$): 3425(NH), 1749(C=O).
$^1$H-NMR 300 MHz $CDCl_3$ chemical shift (ppm, TMS): 5.89 (s broad), 4.18(m), 4.10(t), 3.85-3.72(m), 3.24(t), 2.92(dd), 2.56-2.40(m), 1.0-1.50(m), 1.24(d), 0.88(s), 0.08(s).

Intermediate 5

(3S,4R)-3-[(R)-1-(t-butyldimethylsilyloxy)ethyl]-4-[(R)-2'-[(S)-6'-(2-t-butylidimethylsilyloxyethoxy)-1'-oxocyclohexyl]]azetidin-2-one

Intermediate 4 (1g) was dissolved in dimethylformamide (15ml), with t-butyldimethylsilylchloride (230mg) and imidazole (135mg). The reaction mixture was stirred for 16 hours and then quenched with water using an ice bath to control the exothermic reaction. The mixture was extracted with diethyl ether, dried and concentrated under reduced pressure. Flash chromatography using cyclohexane/ethyl acetate 8/2 as eluant gave the title compound (650mg, RF=0.8).
IR, $CDCl_3$ ($cm^{-1}$): 3418(NH), 1760(C=O lactam), 1717 (C=O).
$^1$H-NMR 300 MHz $CDCl_3$ chemical shift (ppm, TMS): 5.73 (bs), 4.19(m), 3.99(m), 3.73(m), 3.42(m), 3.12(m), 2.86(m), 2.24(m), 2.06(m), 1.80-1.50(m), 1.22(d), 0.88-0.87 (s + s), 0.07(m).

Intermediate 6

Allyl(4S,8S,9R,10S,12R)-4-[2(tosyl)ethoxy]-10-1-(t-butyldimethylsilyloxy)ethyl]-11-oxo-1-azatricyclo-[7.2.0.0.$^{3,8}$]undec-2-ene-2-carboxylate

To a solution of Example 8 (300mg) in dry dichloromethane (15ml) , were added p-tolysulphonyl chloride (0.25g) and triethylamine (0.2ml). The mixture was stirred for 16 hrs at room temperature. Then the solution was diluted with ethyl acetate (100ml). then first washed with a 2.5% aqueous solution of NaHCO3 (2x25ml), then with brine. The solution was dried, the solvent was evaporated off and the crude oil obtained was purified by chromatography on silica gel using a mixture of diethylether and petroleum (1:1) as eluant, obtaining after evaporation of the appropriate fractions, the title compound (130mg) as a oil
IR, $CDCl_3$ $V_{max}$ ($cm^{-1}$): 1776 (C=O), 1717 (C=O), 1632(C=C), 1599(C-C)
$^1$H-NMR s($CDCl_3$) 7.79(d), 7.34(d), 6.01-5.87(m), 5.43(m), 5.25(m), 5.01(t), 4.81-4.61(m), 4.26-4.06(m), 3.53-(t), 3.24-3.10(m), 2.44(s), 1.97(m), 1.9-1.5(m), 1.5-1.2(m), 1.22(d), 0.89(s), 0.091(s), 0.081(s).

Intermediate 7

(3S,4R)-3-[(R)-1-(t-Butyldimethylsilyloxy)ethyl]-4-((R)-2'-(1'-oxocyclohexyl)]azetidin-2-one and (3S,4R)-3-[(R)-1-(t-Butyldimethylsilyloxy)ethyl]-4-((S)-2'-(1'-oxocyclohexyl)]azetidin-2-one

1-Trimethylsilyloxycyclohexene (11g) was dissolved in methylene chloride (400ml) under nitrogen. (3R,4R)-4-Acetoxy-3((R)- (t-butyldimethylsilyloxy)ethyl)-2-azetidinone (9.28g) was added to the solution, the mixture stirred at 23$^0$ and trimethylsilyl trifluoromethanesulphonate (0.66g) was added. The mixture was stirred under nitrogen for 2hr and then poured into an ice cold 1% solution of sodium hydrogen carbonate (300ml). The organic layer was separated, washed with water (300ml) and brine (300ml). Evaporation of the solvent under reduced pressure give the title compounds as an oily residue.

Intermediate 8

(3S,4R)-3-[R)-1-(t-butyldimethylsilyloxy)ethyl]-4-[[(R)-1'-(4-methylphenylsulphono)hydrazono]-cyclohex-2'-yl]-azetidin-2-one(8a) and (3S,4R)-3-[R)-1-(t-butyldimethylsilyloxy)ethyl]-4-[[(S)-1'-(4-methylphenylsulphono)-

hydrazono]-cyclohex-2'-yl]-azetidin-2-one(8b)

To a solution of intermediate 7 (12.1g) in glacial acetic acid (120ml) tosylhydazide (6.9g) was added at room temperature. The reaction was stirred for 3hr, diluted with dichloromethane (250ml), washed with brine (2x250ml), then with a 5% solution of sodium hydrogen carbonate until pH 7, and with brine again (2x150ml). The organic layer was dried and the solvent evaporated under reduced pressure. The obtained foam was stirred with diethyl ether (60 ml) for 2 hr at room temperature filtered and dried to give the title compound 8b as a white powder, (6 g; m.p. 187-189°C; t.l.c. diethyl ether Rf=0.13). IR ($CDCl_3$) $V_{max}$ - ($CM^{-1}$) 3416(N-H), 3304($NNHSO_2$), 1753 (lactam), 15599(C=N; C=C) H[1]-NMR ($CDCl_3$): 7.80 (d) 7.38 (bm), 7.34(d), 5.65 (bs), 4.15 (m) 3.58 (dd) 2.63(m), 2.62(m), 2.44(s), 2.3(m), 2.08(m), 1.92(m), 1.78(d), 1.4(m), 1.20(m), 1.185(d), 0.9(s), 0.077(s), 0.067(s).

The organic layer, which contained the title compound 8a in presence of a small amount of the title compound 8b (by t.l.c.), was concentrated and the residue was purified by flash chromatography (eluant diethyl ether/petroleum ether 7:3) to give the title compound 8a as a white powder (7.6 g; m.p. 95-96°C; t.l.c. diethyl ether Rf-0.37)

IR ($CDCl_3$)$V_{max}$ ($cm^{-1}$) 3410(N-H), 3306($NNHSO_2$), 1755(lactam), 1599 (C-N; C=C) H[1]-NMR ($CDCl_3$): 7.81-(d), 7.40(m), 7.33(d), 5.60(bs) 4.09(m) 4.00(m), 2.81(dd), 2.52(m), 2.44(s), 2.3(m), 2.0-1.8(m), 1.6-1.4(m), 1.04(d) 0.87(s) 0.06(s), 0.03(s).

Intermediate 9

(3S,4R)-3-[R)-1-(t-butyldimethylsilyloxy)ethyl]-4-[[(S)-cyclohex-3'-yl] azetidin-2-one

A solution of the intermediate 8a (1.12g) in anhydrous tetrahydrofuran (20ml) was slowly added, at -40°C, to a stirred solution of diisopropylamide (prepared from anhydrous diisopropylamine (1.35ml) and a 1.6M solution of n-butyllithium in hexane (5.7)). The reaction was slowly warmed to -20°/0°C and maintained at -20°/0°C for 1h. The reaction mixture was added to a precooled 5% solution of hydrochloric acid (20ml) and extracted with ethyl acetate (2x40ml). The organic layer wash washed with a 5% solution of sodium hydrogen carbonate (20ml) and brine (20ml), then dried over anhydrous sodium sulphate and evaporated. The crude product was purified by flash chromatography (eluant diethyl ether/petroleum ether 1/1) to give the title compound as a white powder (0.45g, m.p. 104-06°C; t.l.c. diethyl ether Rf=0.73) IR ($CDCl_3$) $V_{max}$ - ($CM^{-1}$) 3416(N-H), 1753 (lactam), 1603(C=C)

H[1]-NMR ($CDCl_3$): 5.82(bs),. 5.81(m), 5.60(dd), 4.14(m), 3.46(ddO, 2.85(m), 2.24(m), 2.00(m), 1.85-1.70(m), 1.54(m), 1.27(m) 1.23(d), 0.86(s), 0.064(s), 0.054(s).

Intermediate 10

(3S,4R)-3-[(R)-1-(t-butyldimethylsilyloxy)ethyl]-4-[(1'R,2'S,3'R)-1',2'-epoxycyclohex-3'-yl]-azetidin-2-one (10a) and (3S,4R)-3-[(R)-1- (t-butyldimethylsilyloxy)ethyl]-4-[(1'S,2'S,3'R)-1,'2'-epoxycyclohex-3-yl]-azetidin-2-one (10b)

A solution of metachloroperbenzoic acid (3.76g; assay 55%) in dichloromethane (50ml) was added dropwise, at 0°C, to a solution of intermediate 9 (3.1g) in methylene chloride (50 ml). The solution was warmed to room temperature and stirred for 3 hr. The reaction mixture was added to a 10% solution of sodium sulphite (50ml), washed with a 5% solution of sodium hydrogen carbonate (2x50ml) and brine (50ml). The solution was dried over anhydrous sodium sulphate and the solvent was evaporated. The crude product was purified by flash chromatography (eluant ethyl acetate/cyclohexane 3/7) to obtain the title compound 10a as a white powder (1.53g; m.p. 134°-136°C; t.l.c. diethyl ether Rf=0.3)IR ($CDCl_3$) $V_{max}$ (-($cm^{-}1$) 3413(N-H), 1757 (lactam); $^1$H-NHR ($CDCl_3$): 5.85(bm), 4.22(m), 3.77(dd), 3.16(t), 3.12(m), 3.01(m), 2.00-1.7(m), 1.55(m), 1.4(m), 1.24(d), 1.22(m), 0.87(s), 0.067(s) and title compound 10b as a white powder (0.56g; m.p. 96-98°, t.l.c. diethyl ether Rf=0.4) IR ($CDCl_3$)$V_{max}$ ($cm^{-1}$) 3414(N-H), 1757(lactam).

Intermediate 11

(3S,4R)-4-[(2'S,6',R)-2'-(3-t-butyldimethylsilyloxypropoxy)-1'-oxocyclohex-6'yl]-3-[(R)-1'-(t-butyldimethylsilyloxy)ethyl]-azetidin-2-one

To Intermediate 10 (6.2g) dissolved in 35ml of 1,3propanediol and 10ml of dichloromethane was added p-toluensulphonic acid (catalytic amount) and the solution was stirred at room temperature for 24h. Ethyl acetate (150ml) was aded, the organic layer washed twice with brine (3x30ml), dried over $Na_2SO4$ and evaporated in vacuo. The residue was dissolved in 25ml of dry dimethylformamide, imidazole (2.4g) and t-butyldimethylsilychloride 5.32g were added at 0°C, under nitrogen atmosphere. The reaction mixture was stirred at room temperature overnight. Ethyl acetate (100ml) was added and the organic layer was washed twice with 5% HCl (2 x2ml) then with brine (3x 30ml), dried over $Na_2SO_4$ and evaporated in vacuo. The crude residue was purified by flash chromatography (cyclohexane/ethyl acetate 8:2 to 6:4, gradient to give (3S, 4R)-3-[(R)-1-(t-butyldimethylsilyloxy)ethyl]-4-[(2'S,6'R)-2-(3-hydroxypropoxy)-1'-hydroxycyclohex-6'yl]-azetidinone. This compound was then dissolved in 100ml of dry $CH_2Cl_2$ and added at -78°C to a mixture of 3.15ml of oxalylchloride and 5.2ml od dry dimethyl sulphoxide followed by 9.2ml of triethylamine. The solution was stirred at -20°C for 2h, then ethyl acetate (100ml) was added and the organic layer washed with HCl 5% (2x30ml) brine (3x30ml), dried over $Na_2SO_4$ and evaporated in vacuo. The crude residue was purified by flash chromatography (cyclohexane/ethyl acetate 95:5 ti 6:4 gradient), to give the title compound (4.9).

IR (nujol)$V_{max}$ ($cm^{-1}$) 3418(NH), 1757(C=O), 1717(C-o).

$H^1$-NMR ($CDCl_3$): 5.9(bs), 4.18(m), 3.98(dd), 3.75-3.62(m), 3.43(m), 3.08(m), 2.87(dd), 2.28-2.18(m), 2.18-1.92(m), 1.76(q), 1.7-1.45(m), 1.25(d), 0.88(s), 0.078(s), 0.064(s), 0.04(s).

Intermediate 12

(3S,4R)-3-[1R-(t-butyldimethylsilyloxy)ethyl]-4-[6'-(2'S-fluoroethoxy)cyclohexan-1'S-ol]-azetidin-2-one

To a solution of intermediate 10 (5.8g) in 2-fluoroethanol (10 ml) was added ammonium cerium (IV) nitrate (2.44g). The reaction was stirred for 2hrs and then evaporated under vacuum. The mixture was diluted with dichloromethane (100 ml) and washed with brine (50ml). The organic layer was dried and evaporated to give an oily residue which was purified by flash chromatography using a mixture of cyclohexane and ethyl acetate (1/1) as eluant to afford the title compound (1.5g) as a white solid.

IR (CDCl3) vmax (cm-1): 3350 (OH), 3418 (NH), 1761 (C=O), 1103 (C-F); H-NMR s (CDCl3): 6.21-6.08 (sa), 4.59 (t), 4.43 (t), 4.16 (m), 3.88 (t), 3.84-3.52 (m), 3.63 (dd), 3.49 (m), 2.95 (m), 2.10 (sa), 1.9 (m), 1.8-1.2 (m).

Intermediate 13

(3S,4R)-3-[1R-(t-butyldimethylsilyloxy)ethyl]-4-[6'-(2'S-cyanoethoxy)- cyclohexan-1'S-ol]azetidin-2-one

To a solution of intermediate 10 (6g) in 3-hydroxypropionitrile (50 ml) was added ammonium cerium (IV) nitrate (2.35g). The reaction was stirred for 3 hrs then evaporated under vacuum. The mixture was diluted with ethyl acetate (100ml) and washed with water (3x50 ml) and with brine (50 ml). The organic layer was dried and evaporated to give an oily residue which was purified by flash chromatography using a mixture of cyclohexane and ethyl acetate (1/1) as eluant to afford the title compound (1.5 g) as a colourless oil.

IR vmax (cm-1): 3420 (NH), 2251 (CN), 1752 (C=O); H-NMR s (CDCl3) : 6.04 (sa), 4.16 (m), 3.87 (m), 3.73 (m), 3.60 (m), 3.60 (m), 3.51 (m), 2.96 (dd), 2.58 (t), 2.1 (sa), 1.9 (m), 1.8 (m), 1.4 (m), 1.27 (d), 0.88 (s), 0.08 (s).

Intermediate 14

(3S,4R)-3-[1R-(t-butyldimethylsilyloxy)ethyl]-4-[6'-(2'S-cyanoethoxy)- cyclohexanone]-azetidin-2-one

To a cooled (-78C) solution of oxalyl chloride (2.2ml) in dry dichloromethane (50 ml) was added dropwise methyl sulfoxide (3.6 ml). After 30min at -78 C a solution of intermediate 13 (1.5 g) in dry dichloromethane (50 ml) was added maintaining the temperature below -60 C. After 30min triethylamine (17 ml) was added. After 30 min a saturated solution of ammonium chloride (50 ml) and dichloromethane (50 ml) was added. The organic layer was washed with 5% ice cold hydrochloric acid (2x50ml), brine (50ml), then dried and evaporated under vacuum. The oily residue was purified by flash chromatography using diethyl ether as eluant to afford the title compound (0.52 g) as a colourless oil.

IR (CDCl3) vmax (cm-1): 3155, 3084 (NH), 2253 (CN), 1759 (C=O -lactam), 1717 (C=O); H-NMR s (CDCl3): 5.74 (sa), 4.19 (m), 4.04 (t), 3.74 (t), 3.7-3.5 (m), 3.16-3.08 (m), 2.91 (dd), 2.62 (m), 2.28 (m), 2.2-1.96 (m), 1.8-1.5 (m), 1.22 (d), 0.87 (s), 0.08 (s), 0.07 (s).

Example 1

Allyl(4S,8S,9R,10S,12R)-4-(2-methoxyethoxy)-10((1-(t-butyldimethylsilyloxy)ethyl]-11-oxo-1-azatricyclo-[7.2.0.0.$^{3}$.$^{8}$]undec-2-ene-2-carboxylate (1a) and Allyl (4R,8R,9R,10S,12R)-4-(2-methoxyethoxy)-10((1-(t-butyldimethylsilyloxy)ethyl]-11-oxo-1-azatricyclo[7.2.0.0.$^{3}$.$^{8}$]undec-2-ene-2-carboxylate (1b)

Intermediate 1 was dissolved in anhydrous methylene chloride (25ml). Andydrous potassium carbonate (1.00g), and allyl oxalyl chloride (0.67ml) were added followed by triethylamine (0.6ml). The mixture was stirred at 21 C for 2 hours then allyl oxalyl chloride (o.4ml) and triethylamine (0.4ml) were added. The mixture was stirred until the starting material disappeared (3 hrs). The reaction was filtered, concentrated under reduced pressure and extracted with water and ethyl acetate. The organic layer was washed with a cold 2% solution of HCl (50ml) and with a saturated solution of sodium hydrogen carbonate (50ml). The pale yellow solution was dried and evaporated under reduced pressure to yield an oil which was purified by flash chromatography (CH/EA 9/1). Removal of the solvent gave a residue (1g) which was immediatley dissolved in distilled xylene (20ml) and treated with triethyl phosphite (2.5ml). The mixture was refluxed for 5 hrs then cooled, the solvent was removed and the residue was purified by flash chromagrography (CH/EA, gradient elution from 98/2 to 8/2) to give the title compound 1a (170mg) as a colourless oil (Rf=0.5 CH/EA 8.2). Further elution gave 250mg of title compound (1b) (Rf- 0.45 CH/EA 98/2) colourless oil.

title compound (1a)

IR, $V_{max}$ ($cm^{-1}$): 1774 ($\beta$-lactam), 171(carboxyl), 1649 (double bond), 1634(double bond)
$^{1}$H-NMR ($\delta$ ppm), 5.96(m), 5.43(m), 5.25(m), 5.09(t), 4.73(m), 4.20(m), 3.6-3.5(m), 3.38(s), 3.23(m), 3.18(dd), 2.1(m), 1.85(m), 1.62(m), 1.35(m), 1.22(d), 0.88(s), 0.072(s), 0.067(s)

title compound (1b)

IR, $V_{max}$ ($cm^{-1}$): 1774 ($\beta$-lactam), 1717(carboxyl), 1649 (double bond) $^{1}$H-NMR ($\delta$ ppm), 5.97(m), 5.43(m), 5.26(m), 4.98(t), 4.73(m), 4.17(m), 3.68(dd), 3.6-3.4(m), 3.36(s), 3.2(m), 3.14(dd), 2.12(m), 2.00-1.3(m), 1.24-(d), 0.88(s), 0.72(s), 0.67(s).

Example 2

Allyl(4S,8S,9R,10S,12R)-4-(2-methoxyethoxy)-10-((1-hydroxy)ethyl)-11-oxo-1-azatricyclo-[7.2.0.0.$^{3}$.$^{8}$]undec-2-ene-2-carboxylate

Example 1a (170mg) was dissolved in 10ml of anhydrous tetrahydrofuran. Glacial acetic acid (0.17ml) was added followed by a 1M solution of tetrabutylammonium fluoride in tetrahydrofuran (1.2ml). The resulting mixture was stirred for 24 hrs, evaporarated under redcued pressure and extracted with water and ethyl acetate, the organic layer was washed with a cold 2% solution of HCl (30ml), then with a saturated solution of sodium hydrogen carbonate (30ml), dried and evaporated under reduced pressure to give an oil which was chromatrographed (cyclohexane/ethylacetate gradient elution from 8/2 to 3.7) to give the title compound (60mg) (Rf=0.3 CH/EA 2.8).
IR, $V_{max}$ ($cm^{-1}$): 3611(hydroxyl), 1772($\beta$-lactam), 1717(carboxyl)1 1647 (double bond); 1643 (double bond)
$^{1}$H-NMR ($\delta$ ppm), 5.98(m), 5.42(m), 5.09(t), 4.8(m), 4.66(m), 4.23(m), 3.5(m), 3.37(s), 3.3(m), 3.23(dd), 2.11-(m), 2.0-1.55(m), 1.5-1.3(m), 1.3(d).

Example 3

Potassium (4S,8S,9R,10S,12R)-4-(2-methoxyethoxy)-10-((1-hydroxy)ethyl]-11-oxo-1-azatricyclo-[7.2.0.0.$^{3}$.$^{8}$]-undec-2-ene-2-carboxylate

Example 2 (40mg) was dissolved in anhydrous tetrahydrofuran (1ml), a 0.5M solution of potassium 2-ethylhexanoate in ethyl acetate (0.24ml) was added, followed by triphenylphosphine (3mg) and palladium tetrakistriphenhylphosphine (2mg). The resulting mixture was stirred for 30' then diluted with a 1/1 mixture of ethyl ether and petroleum ether (20ml). The white precipitate was filtered, washed twice with petroleum ether and then filtered to give 35mg of the title compound as a white solid which decomposes at 100C.

IR, (Nujol) $V_{max}$ ($cm^{-1}$): 3412(hydroxyl), 1749-1732($\beta$-lactam, broad), 1589 (double bond)
$^1$H-NMR ($D_2O$, ppm) 4.88(m), 4.07(m), 4.01(dd), 3.5-3.3(m), 3.24(m + s), 3.03(m), 1.85(m), 1.70(m), 1.6-1.4-(m), 1.18(m), 1.1(d).

Example 4

Allyl-(4S,8S,9R,10S,12R)-1-aza-4-t-butyldimethylsilyloxyethoxy-10-[1-(2-t-butyldimethylsilyloxy)ethyl]-11-oxo-1-tricyclo [7.2.0.$0^{3,8}$]undec-2-ene-2-carboxylate

Intermediate 5 (650mg), potassium carbonate (250mg) and methylene chloride (15), were placed in a flask and stirred under a nitrogen atmosphere for 5 minutes. Triethylamine (0.3 ml) and allyl oxallylchloride (0.3ml) were added and after three hrs a further 0.2ml of both reactants were added. The mixture was stirred for further 2 hrs, quenched with water and extracted with methylene chloride. The organic layer was washed with hydrochloric acid (2.50ml of 2% solution at 0C), sodium hydrogen carbonate (2x50ml of 5% solution at 0C), dried over anhydrous sodium sulphate and concentrated under reduced pressure. The resultant oil which was dissolved in zylene (20ml), hydroquinone (20mg), triethylphosphite (1.2ml) were added and the mixture was refluxed for 5 hrs. The reaction mixture was cooled at room temperature and concentrated under reduced presssure. The title compound was isolated by flash chromatography using cylcohexane/ethyl acetate as eluant (500mg, RF-0.8).
IR, $CDCl_3$ ($cm^{-1}$): 1769(C-O lactam), 1744(c = O).
$^1$H-NMR 300 MHz $CDCl_3$ chemical shift (ppm, TMS): 7.70(m), 7.50(m), 5.96(m), 5.50-5.30(m), 5.06(t), 4.80-(m), 4.19(m), 4.10(dd), 3.80-3.70(m), 3.42(m), 3.18(dd), 2.06(m), 1.95-1.20(m), 1.22(d), 0.88(m), 0.06(m).

Example 5

Allyl-(4S,8S,9R,10S,12R)-1-aza-4-(2-hydroxyethoxy)-10-[1-(hydroxy)ethyl]-11-oxo-tricyclo[7.2.0.$0^{3,8}$]undec-2-ene-2-carboxylate

Example 4(500mg), tetrahydrofuran (10ml) glacial acetic acid (24ml) tetrabutylammonium fluoride 1 M in tetrahdydrofuran (5ml) were mixed at room tempeature under a nitrogen atomosphere for 24 hrs. The reaction mixture was quenched with water and extracted with ethyl acetate. The organic layer was washed with small amounts of 2% hydrochloridic acid/brine at 0C, 3% sodium hydrogen carbonte at 0C, dried, and concentrated under reduced pressure. Flash chromatography using cyclohexane/ethyl acetate 80/20 to 10/90 as eluant gave the title compound (90mg, RF = 0.3).
IR, $CDCl_3$ ($cm^{-1}$): 1772(C = O lactam), 1716 (C = O).
$^1$H-NMR 300 MHz ($CDCl_3$) chemical shift (ppm, TMS) 7.70-7.53(m), 5.97((m), 5.44(m), 5.26(m), 5.13(t), 4.81-(m), 4.66(m), 4.24(m), 4.19(dd), 3.70(m), 3.47(m), 3.47(m), 3.31-3.20(m), 2.2-1.2(m).

Example 6

Allyl-(4S,8S,9R,10S,12R)-1-aza-4-(2-azidoethoxy)-10-[1-hydroxy)ethyl]-11-oxo-tricyclo[7.2.0.$0^{3,8}$]undec-2-ene-2-carboxylate

To a solution of Example 5 (850mg) dissolved in dry dimethylformamide (10ml) were added sodium azide (814mg) and triphenylphosphine (670mg) followed by carbon tetrabromide (847g) portionwise over 1h at $5^0$C under nitrogen atmosphere. The reaction mixture was stirred at room temperature for 1hr. Ethyl acetate (50ml) was added and the organic layer washed with brine (3x5ml), dried, and evaporated in vacuo. The crude residue was purified by flash chromatography (cyclohexane/ethyl acetate 8:2 to 1:9, gradient) to give the title compound (300mg).
IR(nujol) $V_{max}$ ($cm^{-1}$): 3609(OH), 2108($N_3$), 1780(c = O), 1717(C = O).
$^1$H-NMR ($CDCl_3$): 5.97(m), 5.35(m), 5.12(t), 4.74(m), 4.23(m), 4.20(dd), 3.55(t), 3.5(m), 3.25 (m + dd), 2.0-1.4-(m), 1.32(d).

Example 7

Potassium-(4S,8S,9R,10S,12R)-1-aza-(2-azidoethoxy)-10-[1-(hydroxy)ethyl]-11-oxo-tricyclo[7.2.0.$0^{3,8}$]undec-2-ene-2-carboxylate

To a solution of Example 6 (147mg) in dry dimethylformamide (2ml), were added 0.78ml of a 0.5M solution of potassium-ethylhexanoate in ethyl acetate, triphenylphospine (10ml) and 10mg of $Pd(PPh_3)_4$. The reaction mixture was stirred at room temperature under nitrogen atmosphere for 15min. The solvent was evaaporated in vacuo and the residue was treated with diethyl ether (4ml). The solid compound was filtered and washed twice with diethyl ether to give the title compound 116mg.
IR(nujol) $V_{max}$ ($cm^{-1}$): 2106($N_3$), 1791(C = )), 1591 (C = C. C = O).
$^1$H-NMR ($D_2O$): 4.96(m), 4.07(m), 4.03(dd), 3.6-3.2(m), 3.06(m), 1.87(m), 1.22(m), 1.10(d).

Example 8

Allyl(4S,8S,9R,10S,12R)-4-(2-hydroxyethoxy)-10((1-(t-butyldimethylsilyloxy)ethyl]-11-oxo-1-azatricyclo-[7.2.0.0.$^{3,8}$]undec-2-ene-2-carboxylate

Example 4 (1.15g) was dissolved in tetrahydrofuran (100ml), then acetic acid (0.6g) was added followed by a 1.1M solution of tetrabutylammonium fluoride in tetrahydrofuran (2.83ml). The mixture was stirred at room temperature for 4hrs then diluted with ethyl acetate (100ml). The mixture was washed with a 2.5% aqueous solution of $NaHCO_3$ (2x 50ml), then with brine (2x50ml), the organic layer was dried, evaporated to obtain a crude oil that was purified by chromatography on silica gel suing diethylether/petrol 1/1 as eluant to give the title compound (0.5g) as a colourless oil
IR, ($CECl_3$) $V_{max}$ ($cm^{-1}$): 1775 (C = O)
$^1$H-NMR ($CDCl_3$) 5.97(m), 5.43-5.26(m), 5.13(t), 4.77-4.69(m), 4.24(m), 4.19(dd), 3.73(m), 3.49(m), 3.2-(m + dd), 2.2-1.4(m), 1.23(d), 0.88(s), and 0.08(s) ppm.

Example 9

Allyl(4S,8S,9R,10S,12R)-4-[2-(iodo)-ethoxy-1-10-[(1-(t-butyldimethylsilyloxy)-ethyl]-11-oxo-1-azatricyclo-[7.2.0.0.$^{3,8}$]undec-2-ene-2-carboxylate

To a solution of Intermediate 6 (400mg) in acetone (30ml) was added sodium iodide (300mg) then the mixture was refluxed for 6 hrs. The solvent was then evaporated off and the residue was dissolved using a mixture of dichloromethane (150 ml) and water (150ml). The organic layer was separated, decolourised using activated carbon. Then the suspension was filtered to remove the excess carbon and the obtained solution was evaporated to give the title compound (0.35g) as a white foam.
IR, ($CDCL_3$) $V_{max}$ ($cm^{-1}$): 1772 (C = )), 1717 (C = O), 1647(C = C)
$^1$H-NMR s ($CDCl_3$) 6.03-5.88(m), 5.44(m), 5.27(m), 5.14(t), 4.84-4.64(m), 4.21(m), 4.15(dd), 3.63(m), 3.38-3.2-(m), 3.2(dd), 2.18(m), 2.09(m), 1.7-1.3(m), 1.23(d), 0.88(s), 0.083(s), 0.077(s).

Example 10

Allyl(4S,8S,9R,10S,12R)-4-[2-(iodo)-ethoxy)-10-(1-hydroxy-ethyl-11-oxo-1-azatricyclo-[7.2.0.0$^{3,8}$]undec-2-ene-2-carboxylate

Example 9 (0.2g) was dissovled in tetrahydrofuran (20ml), then acetic acid (0. 5ml) was added followed by a 1.1M solution of tetrabutylammonium fluroide in tetrahydrofuran (1.02ml). The mixture was stirred at room temperature for 25hrs then with ethyl acetate (100ml) was added. The mixture was washed with a 2.5% aqueous solution of $NaHCO_3$ (2x 50ml), then with brine (2x 50ml), the organic layer was dried, evaporated to obtain a crude oil which was purified by chromatography on silica gel using diethylether as elutant. Evaporation of the appropriate fractions gave the title compound (80mg).
IR, ($CDCl_3$) $V_{max}$ ($cm^{-1}$): 3660(OH), 1776 (C = O), 1720(C = O), 1632 (C = C)
$^1$H-NMR s ($CDCl_3$) 6.04-5.90(m), 5.5-5.25 (m), 5.14(t), 4.88-4.64(m), 4.3-4.18(m), 4.21(dd), 3.7-3.54(m), 3.38-(m), 3.3-32(m), 2.1(m), 2.0-1.2(m), 1.32(d).

Example 11

Sodium (4S,8S,9R,10S,12R)-4-[2-(iodo)-ethoxy-]10-(1-hydroxy-ethyl)-11-oxo-1-azatricyclo-[7.2.0.0.$^{3,8}$]undec-2-ene-2-carboxylate

To a solution of Example 10 (33mg) in dry dichloromethane (2ml) was added a solution of triphenyl

phosphine (3.5mg) tetrakis (triphenylphosphine)paddadium (4.6mg) and a 0.5M solution of sodium 2-ethylhexanoate in ethyl acetate (0.142ml) in dichloromethane (1ml). The reaction mixture was stirred for 45 min, then the solution was concentrated to a half volume using a stream of nitrogen. The mixture was then diluted with diethylether and the obtained white solid which precipitated was centrifuged, washed with diethylether (3x 4ml) then dried under vacuum to give the title compound (8mg).
IR, (nujol) $V_{max}$ ($cm^{-1}$): 1751 (C=O), 1597 (C=C)
$^1$H-NMR s ($D_2O$) 4.95 (t), 4.07(m), 4.04(dd), 3.6-3.5(m), 3.26(dd), 3.25-3.1(m), 1.9-1.1(m), 1.10(d).

Example 12

Allyl(4S,8S,9R,10S,12R)-4-[2-(trimethylammonium)-ethoxy]-10-(1-(hydroxy)-ethyl]-11-oxo-1-azatricyclo-[7.2.0.0.$^{3.8}$]undec-2-ene-2-carboxylate

In a well stoppered flash a solution of Example 10 (130mg) in dry acetonitrile (4ml) saturated with trimethylamine was stirred for 1h at 55°C. The solution was then evaporated and the crude residue dissolved with dichloromethane. The solution was evaporated again to give the title compound (140mg).
IR, ($CDCl_3$) $V_{max}$ ($cm^{-1}$): 1800 (C=O), 1744 (C=O)
$^1$H-NMR s ($CDCl_3$) 6.10(m), 5.40(m), 5.30(m), 5.16(m), 4.9-4.6(m), 4.4(m), 4.25(m), 4.05(m), 3.92-3.8(m), 3.48(s), 3.36(m), 3.23(dd), 2.75(bs), 2.1(m), 1.9(m), 1.18-1.14(m), 1.4-1.2(d+m).

Example 13

(4S,8S,9R,10S,12R)-4-[2-(trimethylammonium)-ethoxy-]-10-[1-(hydroxy)-ethyl]-11-oxo-1-azatricyclo-[7.2.0.0.$^{3.8}$]undec-2-ene-2-carboxylate

To a solution of Example 12 (90mg) in dry dichloromethane (8ml) was added a solution of triphenylphosphine (12mg), tetraksi (triphjenylphosphine)palladium (18 mg) in dichloromethane (2ml). Tributylin chloride (55.38mg) was added and the obtained mixture was stirred for a further 10min. The mixture was then first concentrated to a half volume using a stream of nitrogen, then diluted with diethylether (3x 4 ml) then dried under vacuum to give the title compound (60mg).
IR, (nujol) $V_{max}$ ($cm^{-1}$): 1751 (C=O), 1597 (C=C).
$^1$H-NMR s ($D_2O$) 4.93(t), 4.08(m), 4.04(dd), 3.8-3.5(m), 3.5-3.4(m), 3.31(dd), 3.1-3.0(m), 3.04(s), 1.88(m), 1.8-1.7(m), 1.65-1.3(m), 1.25(m), 1.11(d).

Example 14

Potassium-(4S,8S,9R,10S,12R)-1-aza-4-(2-hydroxyethoxy)-10-[1-(hydroxy)-ethyl]-11-oxo-1-azatricyclo-[7.2.0.0.$^{3.8}$]undec-2-ene-2-carboxylate

Example 5 (90mg), palladium tetrakistriphenylphosphine (2mg), triphenylphosphine (3mg) 0.5M potassium-2-ethylhexanoate in ethyl acetate (0.5ml) were dissolved in tetrahydrofuran (2.5ml) and stirred for 20 mintues. Diethyl ether was added and a waxy solid precipitated. Demineralized water (1ml) was added and the mixture was extracted 4 times. The aqeuous solutions were freeze dried and the residue was purified by HPLC (lichrosorb C18 S5, 25x2.0cm, $H_2O/CH_3CN$96/4) yielding the title compound (5mg).
IR, $CDCl_3$ ($cm^{-1}$): 3300-2700(OH), 1749(C=O lactam), 1591(C=O,C=C). .
$^1$H-NMR 300 MHz, $CDCl_3$, chemical shift (ppm, TMS): 4.90(t), 4.07(m), 4.03(dd), 3.65-3.30(m), 3.26(DD), 3.06(m), 1.87(m), 1.80-1.10(m), 1.20(m), 1.10(d).

Example 15

Allyl(4S,8S,9R,10S,12R)-4-(3-t-butyldimethylsilyloxypropanoxy)-10-[1-(t-butyldimethylsilyloxyethyl)]-11-oxo-1-azatricyclo[7.2.0.0.$^{3.8}$]-undec-2-ene carboxylate

To 1.8ml of allyloxalylchloride at 0°C dissolved in 30 ml of dry dichloromethane was added dry triethylamine 1.86ml, under nitrogen atmosphere. After 5 min 4.9g of intermediate 11 dissolved in 30ml of dry methylene were added dropwise over 10min. The reaction mixture was stirred for 30min then ethyl acetate (100ml) was added and the organic layer washed twice with HCl 5% (2 x 20ml), brine (3 x 30ml), $NaHCO_3$ 5% (2 x20ml) then with brine (3 x 30ml), dried over $Na_2SO_4$ and evaporated in vacuo. The residue

which was dissolved in 50ml of dry o-xylene, triethylphosphite (5.96ml) was added followed by catalytic amount of hydroquinone. The reaction mixture was refluxed over nitrogen atmosphere for 4h. The solvent was evaporated in vacuo and the residue purified by flash chromatography (cyclohexane/ethyl acetate 98:2 to 92:8 gradient) to give the title compound 2.52g.
IR (nujol)V $_{max}$ ($cm^{-1}$) 1772(C = O), 1717(C = O), 1650-1630(C = C).
$H^1$-NMR ($CDCl_3$): 6.02-5.88(m), 5.42(m), 5.25(m), 5.03(t), 4.82-4.62(m0, 4.21(m), 4.12(dd), 3.69(m), 3.42(m), 3.84-3.12(m), 3.18(dd), 2.1-2.0(m), 1.96-1.75(m), 1.8-1.7(m), 1.7-1.55(m), 1.5-1.22(m), 1.22(d), 0.88(s), 0.87-(s).

Example 16

Allyl(4S,8S,9R,10S,12R)-4-(3-hydroxypropanoxy)-10-[1-(hydroxyethyl)]-11-oxo-1-azatricyclo[7.2.0.0.$^{3.8}$]-undec-2-ene carboxylate

To Example 15 (2.52g) dissolved in 150ml of dry tetrahydrofuran was added at room temperature a solution of 0.933ml of glacial acetic acid and 13.5ml of a 1.1M solution of tetrabuylammoniumfluoride in tetrahydrofuran. The reaction mixture was stirred at room temperature for 48h then ethyl acetate (250ml) was added and the organic layer washed with HCl 5% ( 2x 20ml) then with brine (3 x 30ml), dried over $Na_2SO_4$ and evaporated in vacuo to give the title compound (0.83g).
IR (nujol)V $_{max}$ ($cm^{-1}$) 3614(OH), 3528-3500(OH), 1774 (C = O), 1720 (C = O), 1649-1634 (C = C).
$H^1$-NMR ($CDCl_3$): 6.04-5.9(m), 5.43(m), 5.27(m), 5.09(m), 4.88-4.62(m), 4.22(m), 4.20(dd), 3.78(bt), 3.54(t), 3.24(dd), 3.3-3.19(m), 2.4-2.3(bs), 2.12-2.0(m), 1.94-1.74(m), 1.7-1.55(m), 1.55-1.3(m), 1.31(m).

Example 17

Allyl(4S,8S,9R,10S,12R)-4-(3-azidopropanoxy)-10-[1-hydroxyethyl)]-11-oxo-1-azatricyclo[7.2.0.0.$^{3.8}$]-undec-2-ene carboxylate

To 0.72g of Example 16 dissolved in 10ml of dry dimethylforamide 0$^0$C under nitrogen atmosphere 0.66g of sodium azide, 0.52g of triphenylphosphine, and 0.66g of carbon tetrabromide dissolved in 4ml of dry dimethyl formamide were added over 15 min. The reaction mixture was stirred at room temperature for 30 min, ethyl acetate (50ml) was added and the organic layer washed with brine (3 x 10ml), dried over $Na_2SO_4$ and evapaorated in vacuo. The crude residue was purified by flash chromatography (cyclohexane/ethyl acetate 8:2 to 6:4 gradient), to give the title compound (0.53g).
IR (nujol)V $_{max}$ ($cm^{-1}$) 3600 (OH), 2100($N_3$), 1780 (C = O), 1720(C = O), 1640(C = C).
$H^1$-NMR ($CDCl_3$): 6.04-5.9(m), 5.44(m), 5.28(m), 5.06(t), 4.88-4.79(m), 4.72-4.64(m), 4.3-4.2(m), 4.19(dd), 3.42-3.34(m), 3.25(dd),3.3-3.16(m), 2.05(m), 1.92-1.78(m), 1.7-1.6(m), 1.88-1.8(m), 1.54-1.32(m), 1.32(d).

Example 18

(4S,8S,9R,10S,12R)-4-(3-azidopropoxy)-10-[1-hydroxyethyl)]-11-oxo-1-azatricyclo[7.2.0.0.$^{3.8}$]-undec-2-ene carboxylic acid sodium salt To 0.53 g of Example 17 dissolved in 8ml of dry tetrahydrofuran were added at room temperature under nitrogen atmosphere 2.7ml of a 0.5M solution of sodium ethylhexanoate in ethyl acetate 35.4mg of triphenyl phosphine and 34.6mg of $Pd(PPh3)_4$. After 20 min the tetrahydrofuran was evaporated in vacuo and the crude residue was precipitated by adding diethyl ether (8ml). The solid compound was filtered and washed twice with diethyl ether to give the title compound(0.42g).
IR (nujol)V $_{max}$ ($cm^{-1}$) : 2097(N3), 1751(C = O), 1595(C = C).
$H^1$-NMR ($CDCl_3$): 4.87(m), 4.07(m), 4.02(dd), 3.4-3.22((m), 3.25(dd), 3.1-2.94(m), 1.85(m), 1.8-1.65(m), 1.11-(d).

Example 19

Allyl (4S,8S,9R,10S)-10-[1R-t-butyldimethylsilyloxy)ethyl]-4-(fluoroethoxy)-11-oxo-1-azatricyclo-[7.2.0.03.8]-undec-2-ene-2-carboxylate

To a cooled (-78 C) solution of oxalyl chloride (0.83 ml) in dry dichloromethane (50 ml) was added dropwise methyl sulfoxide (1.37 ml). After 30min at -78C a solution of intermediate 12 (1.5 g) in dry dichloromethane (50 ml) was added maintaining the temperature below -60 C. After 30min triethylamine (5.4 ml) was added.

After 30min a saturated solution of ammonium chloride (50 ml) and dichloromethane (50ml) was added. The organic layer was washed with 5% ice cold hydrochloric acid (2x50 ml), brine (50 ml), then dried and evaporated under vacuum. The oily residue was purified by flash chromatography using a mixture of cyclohexane and ethyl acetate (6/4) as eluant to afford the ketone (1.37g) which was dissolved in dry dichloromethane (50 ml); then pottassium carbonate (0.5 g) was added. After 10min allyl oxalylchloride (0.95 ml) and triethylamine (0.80 ml) were added. The reaction was stirred for 3hrs then filtered on Celite, diluted with dichloromethane (50 ml). The organic layer was washed with a saturated solution of sodium hydrogen carbonate (50 ml), brine (50ml), then dried and evaporated to give a crude brown oil. The latter was dissolved in dry xylene (100ml); triethylphosphite (3.25 ml) and a catalytic amount of hydroquinone were added. The mixture was stirred at 120° for 5 hrs then the solvent was evaporated and the crude oil was purified by flash chromatography using a mixture of cyclohexane and ethyl acetate (9/1) as eluant to give the title compound (0.77 g) as a brown oil.

IR (CDCl3) vmax (cm-1): 1769 (C=O -lactam), 1744 (C=O) H-NMR s (CDCl3): 5.96 (m), 5.36 (m), 5.14 (t), 4.8-4.7 (m), 4.64-4.47 (m), 4.23 (m), 4.16 (dd), 3.68 (m), 3.58 (m), 3.28 (m), 3.22 (dd), 2.1 (m), 2.0 (m), 1.4 (m), 1.24 (d), 0.92 (s), 0.099 (s).

Example 20

Allyl (4S,8S,9R,10S)-10-[1R-hydroxyethyl]-4-(fluoroethoxy)-11-oxo-1-azatricyclo-[7.2.0.03.8]-undec-2-ene-2-carboxylate

To a solution of Example 19 (0.77g) in dry tetrahydrofuran (50 ml) were added acetic acid (0.76ml) and tetrabutylammonium fluoride (9 ml of a 1M solution in tetrahydrofuran). The mixture was stirred at room temperature for 24 hrs., diluited with ethyl acetate (100 ml) and washed with 2% cold hydrochloric acid (2x50ml), then with a saturated solution of sodium hydrogen carbonate (2x50 ml). The organic layer was dried and evaporated to give an oil which was purified by flash chromatography using a mixture of cyclohexane and ethyl acetate (6/4) as eluant to afford the title compound (0.100g) as a yellow oil.

IR (CDCl3) vmax (cm-1): 1776 (C=O -lactam), 1711 (C=O), 1649 (C=C) H-NMR s (CDCl3): 5.95 (m), 5.35 (m), 5.12 (t), 4.8 (m), 4.6 (m), 4.4 (m), 4.23 (m), 4.17 (m), 3.65 (t), 3.55 (t), 3.3 (m), 3.24 (dd), 2.1 (m), 2.0 (m), 1.4 (m), 1.31 (d).

Example 21

Sodium (4S,8S,9R,10S)-10-[1R-hydroxyethyl]-4-(fluoroethoxy)-11-oxo-1- azatricyclo-[7.2.0.03.8]-undec-2-ene-2-carboxylate

To a stirred solution of Example 20 (0.100g) in dry tetrahydrofuran (2 ml) in a centrifuge tube under nitrogen triphenylphosphine was added (16 mg). In a separate sample tube a solution of tetrakis(triphenylphosphine)-palladium(0) (16 mg) in dry tetrahydrofuran (1ml) was prepared. To this solution sodium-2-ethylhexanoate (0.5ml of a 0.5M solution in ethyl acetate) was added and the resulting solution was quickly added to the solution of intermediate (4). The mixture was stirred for 2 hrs., then diethyl ether (5ml) and petroleum ether (5ml) was carefully added. The solid precipitate was separated after centrifugation and washed with diethyl ether (10 ml). This procedure was repeated three times. The resulting white solid was dried to afford the title compound (95 mg).

IR (CDCl3) vmax (cm-1): 1780 (C=O -lactam), 1750 (C=O), 1610 (C=C) H-NMR s (CDCl3): 4.93 (t), 4.54 (m), 4.38 (m), 4.08 (m), 4.03 (dd), 3.57 (m), 3.46 (m), 3.28 (dd), 3.05 (m), 1.9 (m), 1.8 (m), 1.2 (m), 1.11 (d).

Example 22

Allyl (4S,8S,9R,10S)-10-[1R-(t-butyldimethylsilyloxy)ethyl]-4-(cyanoethoxy)-11-oxo-1-azatricyclo-[7.2.0.03.8]-undec-2-ene-2-carboxylate

Intermediate 14 (0.500g) was dissolved in dry dichloromethane (30 ml); then potassium carbonate (0.3g) was added. After 10' allyl oxalylchloride (0.31 ml) and triethylamine (0.30 ml) were added. The reaction was stirred for 3 hrs then filtered on Celite, diluted with dichloromethane (30 ml). The organic layer was washed with a saturated solution of sodium hydrogen carbonate (30 ml), brine (30 ml), then dried and evaporated to give a crude brown oil. The latter was dissolved in dry xylene (40ml); triethylphosphite (1.05 ml) and a catalytic amount of hydroquinone were added. The mixture was stirred at 120° for 5 hrs then the solvent

was evaporated and the crude oil was purified by flash chromatography using a mixture of cyclohexane and ethyl acetate (9/1) as eluant to give the title compound (0.11 g) as a brown oil.
IR (CDCl3) vmax (cm-1): 2253 (CN), 1772 (C=O -lactam), 1717 (C=O); H-NMR s (CDCl3): 5.95 (m), 3.53 (m), 5.12 (t), 4.74 (m), 4.21 (m), 4.16 (dd), 3.58 (m), 3.25 (m), 3.2 (dd), 2.59 (m), 2.1 (m), 1.9 (m), 2.0 (m), 1.9 (m), 1.21 (d), 1.2 (m), 0.87 (s), 0.072 (s).

Example 23

Allyl (4S,8S,9R,10S)-10-[1R-hydroxyethyl]-4-(cyanoethoxy)-11-oxo-1-azatricyclo[7.2.0.03.8]-undec-2-ene-2-carboxylate

To a solution of Example 22 (0.11g) in dry tetrahydrofuran (10 ml) were added acetic acid (0.08ml) and tetrabutylammonium fluoride (0.95 ml of 1M solution in tetrahydrofuran). The mixture was stirred at room temperature for 24 hrs diluted with ethyl acetate (50ml) and washed with 5% cold hydrochloric acid (2x25ml), then with a saturated solution of sodium hydrogen carbonate (2x25ml). The organic layer was dried and evaporated to give an oil which was purified by flash chromatography using gradient mixtures of cyclohexane and ethyl acetate (from 6/4 to 1/1) as eluant to afford the title compound (0.05 g) as a yellow oil.
IR (CDCl3) vmax (cm-1): 3477 (OH), 2253 (CN), 1772 (C=O -lactam), 1717 (C=O); H-NMR s (CDCl3): 5.96 (m), 5.36 (m), 5.13 (t), 4.75 (m), 4.24 (m), 4.20 (dd), 3.57 (t), 3.32 (m), 3.26 (dd), 2.59 (m), 2.1 (m), 2.0 (m), 1.4 (m), 1.31 (d).

Example 24

Sodium (4S,8S,9R,10S)-10-[1R-hydroxyethyl]-4-(cyanoethoxy)-11-oxo-1-azatricyclo-[7.2.0.03.8]-undec-2-ene-2-carboxylate

To a stirred solution of Example 23 (0.05g) in dry tetrahydrofuran (2ml) in a centrifuge tube under nitrogen was added triphenylphosphine (7mg). In a separate sample tube a solution of tetrakis(triphenylphosphine)-palladium(0) (7 mg) in dry tetrahydrofuran (1 ml) was prepared. To this solution sodium- 2-ethylhexanoate (0.25 ml of a 0.5M solution in ethyl acetate) was added and the resulting solution was quickly added to the solution of intermediate (5). The mixture was stirred for 2 hrs., then diethyl ether (8 ml) and petroleum ether (2 ml) was carefully added. The solid precipitate was separated after centrifugation and washed with diethyl ether (10 ml).
This procedure was repeated three times. The resulting white solid was dried to afford the title compound - (27 mg).
IR (CDCl3) vmax (cm-1): 3418 (OH), 2253 (CN), 1745 (C=O -lactam); H-NMR s (CDCl3): 4.94 (t), 4.04-4.07 (m), 3.49 (m), 3.28 (dd), 3.1 (m), 2.6 (m), 1.9 (m), 1.2 (m), 1.10 (d).

Pharmacy Example

Dry Powder for Injection

Active ingredient (Compound of Example 13)    538mg per vial

Fill sterile vials with the sterile active ingredient. Purge the vial head space with sterile nitrogen; close the vials using rubber plugs and metal overseals (applied by crimping). The product may be constituted by dissolving in Water for Injection (10ml) or other suitable sterile vehicle for injection shortly before the administration
The antibacterial activity of the compounds of the invention may be readily determined using conventional test procedures. For example the antibacterial activity of the compounds of the invention was determined using a standard microtiter broth serial dilution test. In this test the broth was incubated with approximately $10^5$ colony forming units of the test organism and incubated at $35^0$ for 18 hours in the presence of test compound. Results obtained using the test procedure are given in the table below and are expressed as minimum inhibitory concentrations (MIC) in micrograms/ml.

| Organism | | Test Compound MIC μ/ml | | |
|---|---|---|---|---|
| | | 1 | 2 | 3 |
| S aureus | 663E | 0.2 | 0.25 | 0.12 |
| S faecalis | 850E | 4 | 8 | 1 |
| E coli TEM1 | 1919E | 1 | 1 | 0.5 |
| E cloacae | 3647 | 32 | 1 | 32 |
| C perfigens | 615E | 0.03 | 0.25 | ≤ 0.01 |
| B fragilis | 2017E | 0.06 | 1 | 0.2 |

Test compound 1 is the compound of Example 7
Test compound 2 is the compound of Example 13
Test compound 3 is the compound of Example 11
The compounds of the invention are essentially non-toxic at therapeutically useful doses.

## Claims

1. Compounds of the formula (I)

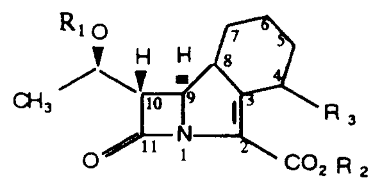

(I)

in which $R_1$ represents a hydrogen atom or a hydroxyl protecting group;
$R_2$ represents a hydrogen atom, a carboxyl protecting group or a cation derived from an inorganic base or an organic base;
$R_3$ represents the group $O(CH_2)_nR_4$ in which n is an integer from 2 to 6 and $R_4$ represents a halogen atom or a hydroxy, $C_{1-3}$alkoxy, $C_{1-3}$ alkythio, azido, cyano, nitro or quaternary ammonium group $N^{+}R_5R_6R_7X^{-}$wherein $R_5$ and $R_6$ independently represent a $C_{1-3}$alkyl group, $R_7$ is a $C_{1-2}$ alkyl group and $X^{-}$ is a pharmaceutically acceptable anion and metabolically labile esters, salts and solvates thereof.

2. Compounds as claimed in Claim 1 wherein $R_1$ and $R_2$ representhydrogen atoms, and physiologically acceptable salts (including internal salts) metabilically labile esters and solvates thereof.

3. Compounds as claimed in Claim 1 or Claim 2 having the configuration

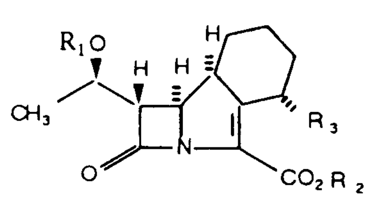

1b

wherein $R_1$, $R_2$ and $R_3$ have the meanings defined above in Claim 1 or Claim 2.

4. Compounds as claimed in any of Claims 1 to 3 wherein $R_4$ represents fluoro, iodo, azido, hydroxy, methoxy, cyano or trimethylammonium.

5. Compounds as claimed in any of Claims 1 to 4 wherein n is 2 or 3.

6. The compounds:
(4S,8S,9R,10S,12R)-4-(2-hydroxyethoxy)-10-(1-hydroxyethyl)-11-oxo-1-azatricyclo [7.2.0.$0^{3,8}$] undec-2-ene-2-carboxylic acid and salts thereof e.g. sodium or potassium salt.
(4S,8S,9R,10S,12R)-4-(2-azidoethoxy)-10-(1-hydroxyethyl)-11-oxo-1-azatricyclo[7.2.0.$0^{3,8}$]undec-2-ene-2-carboxylic acid and salts thereof e.g. potassium or sodium salt.
(4S,8S,9R,10S,12R)-4(2-iodoethoxy)-10-(1-hydroxyethyl)-11-oxo-1-azatricyclo[7.2.0.$0^{3,8}$]undec-2-ene-2-carboxylic acid and salts thereof e.g. potassium or sodium salt.
(4S,8S,9R,10S,12R)-4-[2-(trimethylammonium)-ethoxy]-10-(1-hydroxyethyl)-11-oxo-1-azatricyclo-[7.2.0.$2.^{3,8}$]undec-2-ene-2-carboxylate.
(4S,8S,9R,10S,12R)-4(2-fluoroethoxy)-10-(1-hydroxyethyl)-11-oxo-1-azatricyclo[7.2.0.$0^{3,8}$]undec-2-ene-2-carboxylic acid and salts thereof e.g. potassium or sodium salt.
(4S,8S,9R,10S,12R)-4(2-cyanoethoxy)-10-(1-hydroxyethyl)-11-oxo-1-azatricyclo[7.2.0.$0^{3,8}$]undec-2-ene-2-carboxylic acid and salts thereof e.g. potassium or sodium salt.
(4S,8S,9R,10S,12R)-4(3-hydroxypropoxy)-10-(1-hydroxyethyl)-11-oxo-1-azatricyclo[7.2.0.$0^{3,8}$]undec-2-ene-2-carboxylic acid and salts thereof e.g. potassium or sodium salt.
(4S,8S,9R,10S,12R)-4(3-azidopropoxy)-10-(1-hydroxyethyl)-11-oxo-1-azatricyclo[7.2.0.$0^{3,8}$]undec-2-ene-2-carboxylic acid and salts thereof e.g. potassium or sodium salt.

7. A process for the preparation of compounds as defined in Claim 1 which comprises the cyclisation of a compound of formula (II)

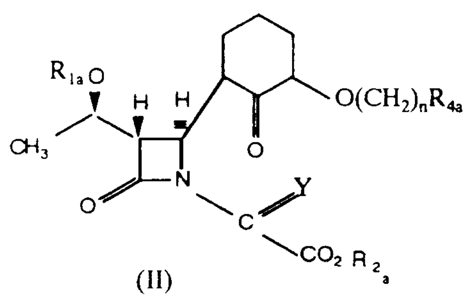

(II)

in which the group $R_{4a}$ has the meansings defined above for $R_4$ or is a group convertible thereto, n is as defined in formula (I) and Y is an oxygen atom or a phosphine group, and the groups $R_{1a}$ and $R_{2a}$ are hydroxy and carboxyl protecting groups as defined for $R_1$ and $R_2$ and if required or desired subjecting the resulting compound prior to or subsequent to any separation into its stereochemical isomers, to one or more of the following operations
(a) removal of one or more protecting groups.
(b) conversion of the group $R_{4a}$ into the group $R_4$.
(c) conversion of a compound in which $R_2$ is a hydrogen atom or a carboxyl protecting group into a salt of an inorganic or organic base.

8. Compounds as claimed in any of Claims 2 to 6 for use in therapy or prophylaxis of systemic or topcial bacterial infections in a human or animal subject.

9. The use of a compound as claimed in any of claims 2 to 6 in the manufacture of a therapeutic agent for the treatment of prophylaxis of systemic or topical bacterial infections in a human or animal body.

10. A method of treatment of a human or non-human body to combat bacterial infections comprising administration of an effective amount of a compound as claimed in any of Claims 2 to 6.

11. Pharmaceutical compositions comprising a compound as claimed in any of Claims 2 to 6 in admixture with one or more physiolgocially acceptable carriers or excipients.

European Patent Office

# PARTIAL EUROPEAN SEARCH REPORT

which under Rule 45 of the European Patent Convention shall be considered, for the purposes of subsequent proceedings, as the European search report

Application Number
EP 92 10 3593

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A | US-A-4 374 848 (B.G. CHRISTENSEN)<br>* Whole document *<br>--- | 1,8-11 | C 07 D 477/00<br>A 61 K 31/40 |
| P,A | EP-A-0 422 596 (TAKEDA CHEMICAL INDUSTRIES, LTD)<br>* Claims *<br>--- | 1,8-11 | |
| P,A | EP-A-0 416 953 (GLAXO S.p.A.)<br>* Claims *<br>--- | 1,8-11 | |
| P,A | EP-A-0 416 952 (GLAXO S.p.A.)<br>* Claims and examples 20-22 *<br>----- | 1,8-11 | |

TECHNICAL FIELDS SEARCHED (Int. Cl.5)

C 07 D
A 61 K

## INCOMPLETE SEARCH

The Search Division considers that the present European patent application does not comply with the provisions of the European Patent Convention to such an extent that it is not possible to carry out a meaningful search into the state of the art on the basis of some of the claims

Claims searched completely :

Claims searched incompletely :

Claims not searched :

Reason for the limitation of the search:

Remark: Although claim 10 is directed to a method of treatment of (diagnostic method practised on) the human/animal body (Article 52(4) EPC) the search has been carried out and based on the alleged effects of the compound/composition.

| Place of search | Date of completion of the search | Examiner |
|---|---|---|
| THE HAGUE | 18-05-1992 | CHOULY J. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0407)